# EUROPEAN PATENT APPLICATION

(11) **EP 1 777 521 A1**
(43) Date of publication of application: **25.04.2007**
(21) Application number: 06122686.6
(22) Date of filing: 20.10.2006
(51) Int. Cl.: G01N 33/569

(54) **Method and kit for the determination of HIV infection**

(30) Priority: 20.10.2005 JP 2005306419; 20.10.2005 JP 2005306420
(71) Applicant: Pentax Corporation, Tokyo 174-8639 (JP)
(72) Inventor: Yagasaki, Kazumi, Itabashi-ku, Tokyo 174-8639 (JP); Miyazaki, Isao, Itabashi-ku, Tokyo 174-8639 (JP)
(74) Representative: Schaumburg, Thoenes, Thurn, Landskron

(57) **Abstract**

Disclosed herein are a determination method capable of determining the presence or absence of an anti-HIV antibody in a sample with accuracy even if an operation of cleaning is omitted and a determination kit to be used for the determination method. The determination method is as follows. First, an antagonist containing a protein having a high affinity for an inhibitory substance than for the HIV antigen is added to a liquid specimen containing a sample obtained from a living body. Then, this composition is fed into a well carrying a mixture of HIV antigens on its inner surface. Next, a reagent containing colored particles carrying an anti-IgG antibody is fed into the well. In a case where the anti-IgG antibody is bound to the anti-HIV antibody which has been bound to the HIV antigens so that the particles are dispersed in the well, the sample is positive. In a case where the particles are precipitated and agglutinated in the well, the sample is negative.

## Description

### BACKGROUND OF THE INVENTION

### Field of the Invention

The present invention relates to a determination method and a determination kit, and more specially relate to a determination method capable of determining the presence or absence of an anti-HIV antibody and a determination kit to be used for the determination method.

Immunoassay based on an antigen-antibody reaction is utilized for diagnosing of various infection diseases, because it is determined a trace constituent in a biological sample specifically and sensitively.

For example, particle agglutination (e.g., see USP 5,540,995), enzyme-linked immunosorbent assay (ELISA), radioimmunoassay (RIA) and immunochromatography are known as the immunoassay.

The particle agglutination (hereinafter, simply referred to as "PA") is advantageous because it is a simple and sensitive method which requires fewer steps and is capable of determining the presence or absence of virus infection in a short period of time without using special devices or equipment.

For this reason, the PA is expected as a determining method for various viruses such as HIV (Human Immunodeficiency Virus) and the like.

For example, a determination of an anti-HIV antibody by using the PA is carried out as the following.

I First, a blood serum sample is fed into a well carrying a HIV antigen on an inner surface thereof.

It is to be noted that in a case where the blood serum sample contains anti-HIV antibodies, these anti-HIV antibodies are bound to the HIV antigen.

II Next, the well is cleaned. By doing so, it is possible to almost remove an accompanying component other than the anti-HIV antibodies which have been bound to the HIV antigen.

III Next, a reagent containing colored particles carrying an anti-IgG antibody, which can be bound to the anti-HIV IgG antibody, is fed into the well.

At this time, in a case where the anti-HIV antibodies have been bound to the HIV antigen, the anti-IgG antibody is bound to the anti-HIV IgG antibody. As a result, the colored particles are adsorbed to the inner surface of the well through the anti-IgG antibodies, the anti-HIV antibodies and the HIV antigens with the colored particles being dispersed in the liquid in the well, and therefore the inside of the well (a liquid contained in the well) is lightly colored. In this case, the blood serum sample is determined to be positive.

On the other hand, in a case where the anti-HIV antibody is not bound to the HIV antigen, since an opponent to which the anti-IgG antibody is to be bound is absent, the colored particles are precipitated and agglutinated in the well. As a result, a highly colored area is formed at a bottom of the well. In this case, the blood serum sample is determined to be negative.

In such a PA, after the blood serum sample is fed into the well, the well is cleaned. This operation of cleaning is carried out for removing the accompanying constituent other than the anti-HIV antibody bound to the HIV antigen from the well. In this connection, since the blood serum sample contains an inhibitory substance which interferes with the aggregation or precipitation of the colored particles, it becomes important to remove such an inhibitory substance from the well.

However, the operation of cleaning the well is cumbersome, which causes a problem that an operating efficiency for determining the anti-HIV antibody is reduced. Further, in a case where the blood serum sample contains HIV, there is a risk that an operator becomes infected with HIV due to a blood serum sample or the like spattering around the operator during the operation of cleaning.

On the other hand, in a case where the operation of cleaning is omitted, when the reagent is fed into the well, the aggregation or precipitation of the colored particles is interfered due to the inhibitory substance. As a result, the colored particles are adsorbed to the inner surface of the well through the anti-IgG antibodies, the inhibitory substances and the HIV antigens with the colored particles being dispersed in the liquid in the well, even if the anti-HIV antibodies are not bound to the HIV antigens. Namely, There is a problem that a negative blood serum sample is misjudged to be positive.

### SUMMARY OF THE INVENTION

It is therefore an object of the present invention to a determination method capable of determining the presence or absence of an anti-HIV antibody in a sample with accuracy even if an operation of cleaning is omitted and a determination kit to be used for the determination method.

In order to achieve the above object, the present invention is directed to a determination method for determining whether or not a living body is infected with HIV (Human Immunodeficiency Virus) using a reagent containing particles carrying an anti-IgG antibody, comprising:
preparing a well carrying a HIV antigen on an inner surface thereof;
feeding a liquid specimen containing a sample obtained from the living body into the well, wherein the sample also containing an inhibitory substance which is to be bound to the HIV antigen and the anti-IgG antibody to interfere with the precipitation of the particles in the well;
feeding the reagent containing the particles into the well; and
determining that in a case where the anti-IgG antibody is bound to the anti-HIV antibody which has been bound to the HIV antigen so that the particles are dispersed in the well, the sample is positive, while that in a case where the particles are precipitated and agglutinated in the well, the sample is negative;
wherein before feeding the liquid specimen into the well, an antagonist containing a protein having an affinity for the inhibitory substance higher than that of the HIV antigen is added to the liquid specimen.

Such a determination method is capable of determining the presence or absence of an anti-HIV antibody in a sample with accuracy even if an operation of cleaning is omitted.

In the determination method of the present invention, it is preferred that the protein is at least one selected from the group comprising casein, ovalbumin, albumin, lactoferrin and chymotrypsinogen.

These proteins are preferred because their affinity for the inhibitory substance is especially higher than that of the HIV antigen.

Further, in the determination method of the present invention, it is also preferred that the particles are colored.

This is advantageous in that the determination of the presence or absence of anti-HIV antibodies can be made under visible light irradiation.

In the determination method of the present invention, it is also preferred that the specific gravity of the particles is larger than that of a liquid present in the well in the determining Step.

By setting the specific gravity of the particles to a value within the above range, it is possible to speedily and reliably precipitate and agglutinate the particles in the well when necessary (i.e., when a liquid specimen should be determined to be negative).

Further, in the determination method of the present invention, it is also preferred that the antagonist further contains a nonionic surfactant.

This makes it possible to effectively interfere with the binding of the inhibitory substance to the HIV antigen or the anti-IgG antibody.

In the determination method of the present invention, it is also preferred that the bottom of the well is made convergent.

This allows the precipitated particles to be rolled toward the central portion of the well and then to be gathered together and agglutinated. As a result, in a case where the particles contained in the reagent are colored, these colored particles can be visually recognized by their hue of a deep color at the central portion of the well (i.e., the central portion of the well is highly colored), thereby facilitating the recognition of precipitation and agglutination (i.e., motion and state) of the colored particles in the well.

In the determination method of the present invention, it is also preferred that the sample is blood serum, and the liquid specimen is prepared by diluting the blood serum 2- to 1000-fold.

According to the present invention, the precipitation inhibitory action of the inhibitory substance is inhibited by the action of the antagonist, by adding the antagonist containing the protein to the liquid specimen before the liquid specimen is fed into the well. Therefore, even when the dilution rate of the sample is such a relatively low value (that is, even when a relatively large amount of the inhibitory substance is present in the liquid specimen), it is possible to determine whether the liquid specimen is negative or positive with accuracy. Further, in the case of such a relatively low dilution rate, the operation of diluting a sample can be relatively easily carried out in a short period of time, thereby reducing the time required for determining the presence or absence of the anti-HIV antibody in a sample.

Another aspect of the present invention is directed to be used for the determination method described above, comprising:
a plate having a plurality of wells each carrying the HIV antigen on an inner surface thereof;
a reagent containing the particles; and
an antagonist containing the protein.

Such a determination kit is capable of determining the presence or absence of an anti-HIV antibody in a sample with accuracy even if an operation of cleaning is omitted.

### BRIEF DESCRIPTION OF THE DRAWINGS

Fig. 1 is a perspective view which schematically shows a plate of a determination kit of the present invention;
Figs. 2(a) and 2(b) are illustrations for explaining a determination method of the present invention;
Fig. 3 is an illustration for explaining the determination method of the present invention;
Fig. 4 is an illustration for explaining the determination method of the present invention;
Fig. 5 is a photograph which shows results determined in the Example 1A and the Comparative Example 1A; and
Fig. 6 is a photograph which shows results determined in the Example 1B to 5B and the Comparative Example 1B to 3B.

### DETAILED DESCRIPTION OF THE INVENTION

Hereinbelow, a determination method and a determination kit according to the present invention will be described in detail with reference to a preferred embodiment shown in the accompanying drawings.

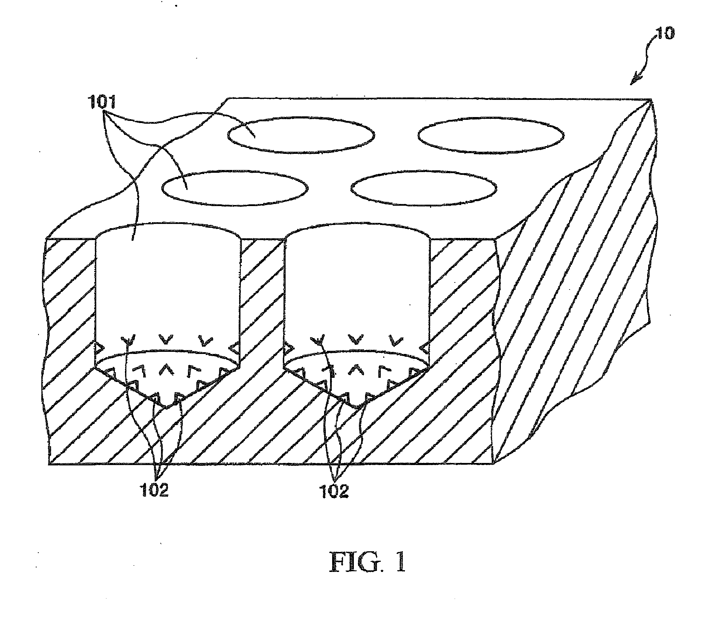

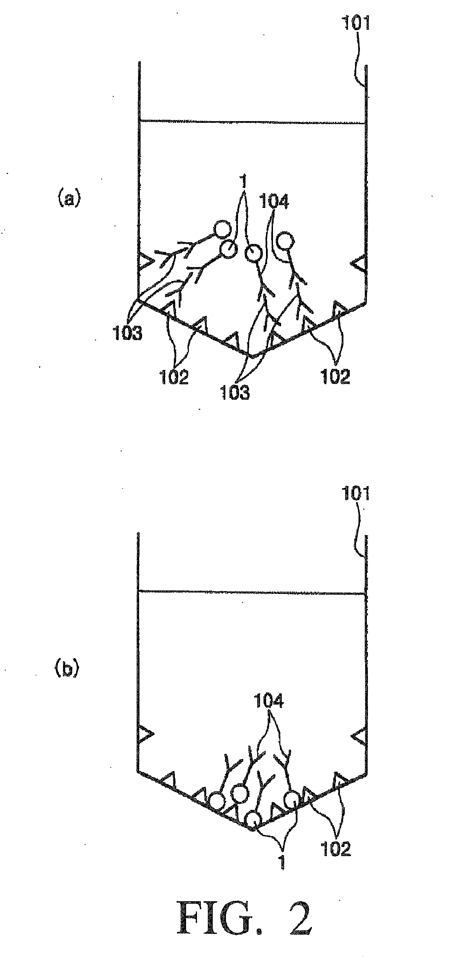

Fig. 1 is a perspective view which schematically shows a plate of a determination kit of the present invention, and Figs. 2 to 4 are illustrations for explaining a determination method of the present invention.

The determination kit of the present invention is used to determine whether or not a living body is infected with HIV (Human Immunodeficiency Virus) by detecting the presence or absence of an anti-HIV antibody in a sample obtained from the living body by utilizing particle agglutination.

In this connection, the detection of an anti-HIV antibody (i.e., a determination as to whether or not a living body is infected with HIV) using particle agglutination is carried out by, for example, the following method.

First, a liquid specimen containing a sample obtained from a living body is fed into a well whose inner surface carries a HIV antigen. Then, a reagent containing colored particles carrying an anti-IgG antibody is fed into the well to determine whether an anti-HIV antibody is present (positive) or absent (negative) in the liquid specimen (i.e., in the sample).

The determination kit of the present invention is used for such determination of the presence or absence of anti-HIV antibodies. The determination kit includes a plate 10 equipped with a plurality of wells 101 each carrying a HIV antigen 102, a reagent (i.e., a reagent for determination) containing colored particles 1 carrying an anti-IgG antibody 104, and an antagonist containing a protein 106 capable of interfering with the binding of an inhibitory substance 105, which interferes with the precipitation of the colored particles 1, to the HIV antigen 102.

As shown in Fig. 1, the plate 10 is formed from a flat plate-shaped member, and has a plurality of wells 101 arranged in a matrix in one of the surfaces thereof.

Examples of a material for forming the plate 10 include resin materials such as polystyrene, polycarbonate, and polypropylene. These resin materials can be used singly or in combination of two or more of them.

The well 101 provides space for holding (storing) the liquid specimen and the reagent, and carries a HIV antigen 102 on the inner surface thereof.

As shown in Fig. 1, the well 101 has a shape such that the bottom thereof is made convergent (i.e., in this embodiment, the depth of the well 101 becomes deeper toward the central portion of the well 101). This allows the precipitated colored particles 1 to be rolled toward the central portion of the well 101 and then to be gathered together and agglutinated. As a result, the colored particles 1 can be visually recognized by their hue of a deep color at the central portion of the well 101 (i.e., the central portion of the well 101 is highly colored), thereby facilitating the recognition of precipitation and agglutination (i.e., motion and state) of the colored particles 1 in the well 101.

As described above, the well 101 preferably has a shape such that the bottom thereof is made convergent. However, unlike the well 101 shown in Fig.1, the deepest point of the well 101 may be off the central portion of the well 101.

The capacity of the well 101 is not particularly limited, but is preferably in the range of about 0.03 to 0.5 mL, and more preferably in the range of about 0.05 to 0.3 mL. By setting the capacity of the well 101 to a value within the above range, it is possible to hold a liquid (i.e., the liquid specimen and the reagent) in an amount necessary and sufficient to make determination of the presence or absence of anti-HIV antibodies without increasing the size of the plate 10.

The HIV antigen 102 is carried on the inner surface of the well 101 by, for example, hydrophobic bonding.

Examples of the HIV antigen 102 include HIV-1 p18, HIV-1 p24, HIV-1 p55, HIV-1 p31, HIV-1 p51, HIV-1 p66, HIV-1 gp41, HIV-1 gp120, HIV-1 gp160, HIV-2 p16, HIV-2 p26, HIV-2 p56, HIV-2 p34, HIV-2 p53, HIV-2 p68, HIV-2 gp36, HIV-2 gp105, and HIV-2 gp140. These HIV antigens 102 can be used singly or in combination of two or more of them.

The amount of the HIV antigen 102 to be carried on the inner surface of the well 101 is preferably 0.01 µg or more, and more preferably in the range of about 0.05 to 1 µg. By setting the amount of the HIV antigen 102 to a value within the above range, it is possible to allow a sufficient amount of an anti-HIV antibody 103 to bind to the HIV antigen 102 when the anti-HIV antibody 103 is present in the sample (i.e., in the liquid specimen), thereby enabling the detection of the anti-HIV antibody 103 to be carried out more accurately. It is to be noted that even if the amount of the HIV antigen 102 is increased to exceed the above upper limit value, it cannot be expected that the accuracy of detection is further improved.

The reagent is a suspension containing colored particles 1 carrying an anti-IgG antibody 104.

When such a reagent is used, as shown in Fig. 2(a), the anti-IgG antibody 104 (i.e., a labeled secondary antibody) carried on the colored particles 1 is bound to an anti-HIV antibody 103 (i.e., a primary antibody) in a case where the anti-HIV antibody 103 has been bound to the HIV antigen 102. As a result, the colored particles 1 are adsorbed to the inner surface of the well 101 through the anti-IgG antibodies 104, the anti-HIV antibodies 103 and the HIV antigens 102 with the colored particles 1 being dispersed in the liquid in the well 101.

On the other hand, as shown in Fig. 2(b), in a case where an anti-HIV antibody 103 is not bound to the HIV antigen 102, the colored particles 1 are precipitated and agglutinated in the well 101.

Namely, after the reagent is fed into the well 101, in a case where the colored particles 1 are adsorbed to the inner surface of the well 101 through the anti-IgG antibodies 104, the anti-HIV antibodies 103 and the HIV antigens 102 with the colored particles 1 being dispersed in the liquid in the well 101, the sample can be determined to be positive (that is, the sample can be determined to contain an anti-HIV antibody 103). On the other hand, after the reagent is fed into the well 101, in a case where the particles are precipitated and agglutinated in the well 101, the sample can be determined to be negative (that is, the sample can be determined to contain no anti-HIV antibody 103).

It is to be noted that in this embodiment, colored particles 1 are used as particles contained in the reagent. However, in the present invention, other particles such as particles carrying a fluorescent material may be alternatively used. The merit of using colored particles 1 as particles contained in the reagent is that the determination of the presence or absence of HIV infection can be made under visible light irradiation.

Such particles (colored particles 1) can be formed using a resin material, a ceramic material, or the like. Alternatively, composite particles composed of a resin material and a ceramic material (e.g., resin particles coated with a ceramic material) may be used.

Examples of the resin material include thermoplastic resins such as polyamide (e.g., nylon 6, nylon 6.6, nylon 6.10, nylon 12), polyethylene, polypropylene, polystyrene, polyimide, methacrylic resins, acrylic resins, and thermoplastic polyurethanes, and thermosetting resins such as epoxy resins, phenol resins, melamine resins, urea resins, unsaturated polyesters, alkyd resins, thermosetting polyurethanes, and ebonite.

Examples of the ceramic material include calcium phosphate-based compounds such as Ca₁₀(PO₄)₆(OH)₂, ca₁₀(PO₄)₆F₂, Ca₁₀(PO₄)₆Cl₂, Ca₃(PO₄)₂, Ca₂P₂O₇, Ca(PO₃)₂, and CaHPO₄, alumina, and silica.

The above-described composite particles can be produced by, for example, colliding ceramic porous particles with the surface of resin particles by the use of a commercially-available hybridization machine or mechanofusion machine.

The specific gravity of the particles is preferably larger than that of a liquid present in the well 101 in Step <4> which will be described later. More specifically, the specific gravity of the particles is preferably in the range of about 1.05 to 1.3 g/cm³. By setting the specific gravity of the particles to a value within the above range, it is possible to speedily and reliably precipitate and agglutinate the particles in the well 101 when necessary (i.e., when a liquid specimen should be determined to be negative).

The average particle diameter of the particles is preferably 30 µm or less, and more preferably in the range of about 1 to 20 µm.

In a case where colored particles 1 are used as particles contained in the reagent, they can be prepared by staining particles with a dye or pigment. Examples of the dye include direct dyes, acid dyes, basic dyes, mordant dyes, acid mordant dyes, sulfur dyes, sulfur vat dyes, vat dyes, soluble vat dyes, azoic dyes, disperse dyes, reactive dyes, oxidation dyes, dyes for synthetic fibers, and fluorescent whitening dyes.

On the surface of the colored particles 1 (particles), an anti-IgG antibody 104 is immobilized (carried) directly or with the aid of an immobilizing agent.

Examples of the immobilizing agent include glutaraldehyde, formaldehyde, coupling agents, and osmium tetrachloride. Examples of the coupling agents include 3-glycidoxypropyl trimethoxysilane, 3-thiopropyl trimethoxysilane, 2-(3-trimethoxysilylpropyldithio)-5-nitropyridine, 3-aminopropyl triethoxysilane, and 3-chloropropyl dimethoxymethylsilane.

In a case where at least the surface of each of the colored particles (colored beads) 1 and its vicinity are made of a calcium phosphate-based compound, part of the surface of each of the colored particles 1 where the anti-IgG antibody 104 is not immobilized is preferably blocked (coated) with a blocking agent after the anti-IgG antibody 104 is immobilized on the surface of each of the colored particles 1. As such a blocking agent, one having no effect on an antigen-antibody reaction, such as casein or albumin, is preferably used.

The amount of the colored particles 1 to be contained in the reagent is not particularly limited, but is preferably in the range of about 0.05 to 0.2 wt%, and more preferably in the range of about 0.08 to 0.12 wt%. By setting the amount of the colored particles 1 to be contained in the reagent to a value within the above range, it is possible to visually recognize the motion and state of the colored particles 1 in the well 101 with reliability. If the amount of the colored particles 1 contained in the reagent is too much, there is a case that some inconveniences, such as clogging of a pipet tip, occur when the reagent is fed (dispensed) into the wells 101.

Examples of a dispersion medium to be used for preparing the reagent include various buffers such as triethanolamine hydrochloride-sodium hydroxide buffer, veronal (sodium 5,5-diethyl barbiturate)-hydrochloride buffer, Tris-hydrochloride buffer, glycylglycine-sodium hydroxide buffer, 2-amino-2-methyl-1,3-propanediol-hydrochloride buffer, diethanolamine-hydrochloride buffer, boric acid buffer, sodium borate-hydrochloride buffer, glycine-sodium hydroxide buffer, sodium carbonate-sodium bicarbonate buffer, sodium borate-sodium hydroxide buffer, sodium bicarbonate-sodium hydroxide buffer, potassium dihydrogen phosphate-disodium hydrogen phosphate buffer, disodium phosphate-sodium hydroxide buffer, potassium chloride-sodium hydroxide buffer, Britton-Robinson buffer, and GTA buffer, and those obtained by adding normal saline to these buffers.

Here, a sample obtained from a living body (especially, blood serum) contains many kinds of inhibitory substances 105 which bind to the HIV antigen 102 and the anti-IgG antibody 104 to interfere with the precipitation of the colored particles 1.

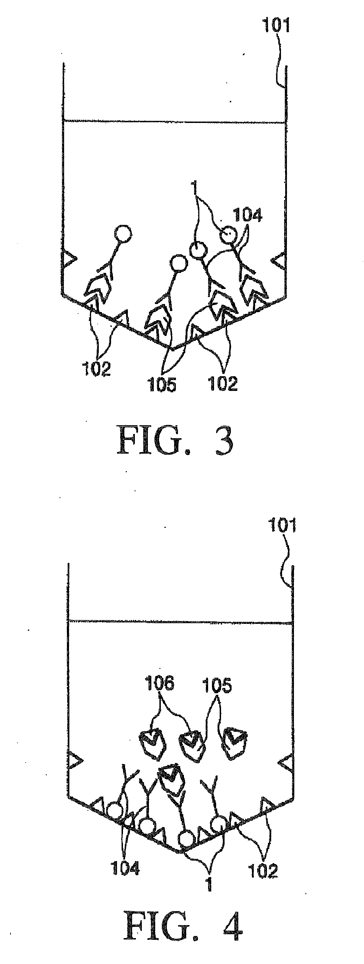

As shown in Fig. 3, in a case where such an inhibitory substance 105 has been bound to the HIV antigen 102 carried on the inner surface of the well 101, the anti-IgG antibody 104 contained in the reagent fed into the well 101 is further bound to the inhibitory substance 105. As a result, the colored particles 1 are adsorbed to the inner surface of the well 101 through the anti-IgG antibodies 104, the inhibitory substances 105 and the HIV antigens 102 with the colored particles 1 being dispersed in the liquid in the well 101. This causes a mistaken determination that a negative liquid specimen (a sample) is misjudged to be positive.

According to the present invention, in order to prevent such a mistaken determination, an antagonist containing a protein 106 having an affinity for the inhibitory substance 105 higher than that of the HIV antigen 102 is added to a liquid specimen before the liquid specimen is fed into the well 101.

By doing so, the protein 106 is selectively (specifically) bound to the inhibitory substance 105 contained in the liquid specimen (i.e., in the sample). This prevents (inhibits) the binding of the inhibitory substance 105 contained in the liquid specimen fed into the well 101 to the HIV antigen 102 because, as shown in Fig. 4, the HIV antigen 102-binding site of the inhibitory substance 105 is blocked by the protein 106.

Therefore, it is possible to prevent the occurrence of a phenomenon in which the anti-IgG antibody 104 is further bound to the inhibitory substance 105 bound to the HIV antigen 102 so that the colored particles 1 are dispersed in the liquid in the well 101 (which indicates that the liquid specimen is positive for HIV), that is, it is possible to reliably precipitate and agglutinate the colored particles 1, thereby reliably determining a liquid specimen containing no anti-HIV antibody 103 to be negative.

The protein 106 is not particularly limited as long as it has an affinity for the inhibitory substance 105 higher than that of the HIV antigen 102 and has substantially no ability to bind to the HIV antigen 102, but is preferably at least one selected from the group comprising casein (phosphate protein), ovalbumin (glycoprotein), albumin, lactoferrin (glycoprotein) and chymotrypsinogen. Particularly, at least one selected from the group comprising casein, ovalbumin, and lactoferrin is preferably used. These proteins are preferred because their affinity for the inhibitory substance 105 is especially higher than that of the HIV antigen 102.

The amount of the antagonist to be added to a liquid specimen is not particularly limited, but is preferably in the range of about 0.1 to 10 mg, more preferably in the range of about 0.3 to 8 mg, and even more preferably in the range of about 0.3 to 3 mg in terms of the amount of the protein 106, per µg of the HIV antigen 102. By setting the amount of the protein 106 to be added to a liquid specimen to a value within the above range, it is possible to reliably reduce the amount of the inhibitory substance 105 not bound to the protein 106, thereby reliably interfering with the action of the inhibitory substance 105 that inhibits the precipitation of the colored particles 1 (hereinafter, simply referred to as "precipitation inhibitory action"). It is to be noted that even if the amount of the protein 106 to be added to a liquid specimen is increased to exceed the above upper limit value, it cannot be expected that the interfering effect of the action of the inhibitory substance 105 obtained by adding the protein 106 is further enhanced.

The protein 106 may be directly used as the antagonist, but it is preferred that the protein 106 is dissolved in an appropriate solvent before use. By doing so, the amount of the protein 106 to be added to a liquid specimen can be easily controlled.

Examples of the solvent include the buffers mentioned above with reference to the preparation of a reagent.

Preferably, the antagonist further contains a nonionic surfactant, which makes it possible to effectively interfere with the binding of the inhibitory substance 105 to the HIV antigen 102 or the anti-IgG antibody 104.

Examples of the nonionic surfactant include Polyoxyethylene(9)-octylphenyl ether, polyoxyethylene(20)-sorbitan-monolaurate, polyoxyethylene(10)-octylphenyl ether, and n-nonanonyl-N-methylglucamide. These nonionic surfactants can be used singly or in combination of two or more of them.

Next, a method for using such a determination kit described above, that is, a method for determining whether or not a living body is infected with HIV (i.e., a determination method of the present invention) will be described.
<1> First, a liquid specimen to be subjected to a determination of the presence or absence of HIV infection (an anti-HIV antibody) is prepared. As such a liquid specimen, a sample obtained from a living body can be directly used. If necessary, the volume of the sample may be adjusted by, for example, dilution before use.
   Examples of a liquid to be used for adjusting the volume of the sample include the buffers mentioned above with reference to the preparation of a reagent.
   Examples of the sample include liquids such as blood serum (blood), semen, vaginal secretion, breast milk, and solids (solid matter) such as cells (tissues). It is to be noted that in a case where a solid sample such as cells is used, a liquid specimen can be prepared by crushing the sample and suspending the crushed sample in the buffer.
   In a case where blood serum is used as a sample, it is preferred that a liquid specimen is prepared by diluting blood serum about 2- to 1000-fold (especially, about 5- to 100-fold). According to the present invention, the precipitation inhibitory action of the inhibitory substance 105 is inhibited by the action of the antagonist, by adding the antagonist containing the protein 106 to the liquid specimen before the liquid specimen is fed into the well 101. Therefore, even when the dilution rate of the sample is such a relatively low value (that is, even when a relatively large amount of the inhibitory substance 105 is present in the liquid specimen), it is possible to determine whether the liquid specimen is negative or positive with accuracy. Further, in the case of such a relatively low dilution rate, the operation of diluting a sample can be relatively easily carried out in a short period of time, thereby reducing the time required for determining the presence or absence of the anti-HIV antibody 103 in a sample.
<2> Next, an antagonist is added and mixed to a liquid specimen.
   By adding the antagonist to the liquid specimen, a protein 106 contained in the antagonist is bound to an inhibitory substance 105.
   It is to be noted that in a case where the antagonist is a liquid (liquid state), the antagonist in the liquid state can be used as a liquid to be used for adjusting the volume of the sample in the above-mentioned step <1>
<3> Next, a plate 10 having well 101 carrying a HIV antigens 102 on the inner surface thereof is prepared. Then, the liquid specimen is fed (supplied) into the well 101.
   In a case where the liquid specimen contains an anti-HIV antibody 103, as shown in Fig. 2(a), the anti-HIV antibody 103 contained in the liquid specimen fed into the well 101 is bound to (captured by) the HIV antigen 102 carried on the inner surface of the well 101.
   At this time, the inhibitory substance 105 tries to be bound to the HIV antigen 102. However, according to the present invention, as shown in Fig. 4, the protein 106 added to the liquid specimen in the step <2> is bound to the inhibitory substance 105. Namely, the HIV antigen 102-binding site of the inhibitory substance 105 is blocked by the protein 106. As a result, the binding of the inhibitory substance 105 to the HIV antigen 102 can be prevented.
   It is to be noted that the amount of the liquid specimen to be fed into each well 101 is not particularly limited, but is preferably in the range of about 0.02 to 0.25 mL, and more preferably in the range of about 0.04 to 0.06 mL.
<4> Next, a reagent is fed into the well 101 and mixed in a liquid specimen in the well 101.

As shown in Fig. 2 (a), in a case where an anti-HIV antibody 103 has been bound to the antigen 102, an anti-IgG antibody 104 carried on colored particles 1 contained in the reagent fed into the well 101 is bound to the anti-HIV antibody 103. As a result, the colored particles 1 are adsorbed to the inner surface of the well 101 through the anti-IgG antibodies 104, the anti-HIV antibodies 103 and the HIV antigens 102 with the colored particles 1 being dispersed in the liquid in the well 101 (i.e. , in a mixture of the liquid specimen and the reagent) so that the liquid contained in the well 101 appears to be lightly colored. In this case, the sample is determined to be positive (that is, the sample is determined to contain an anti-HIV antibody 103).

On the other hand, as shown in Fig. 2(b), in a case where no anti-HIV antibody 103 is bound to the antigen 102, the colored particles 1 carrying the anti-IgG antibody 104 are precipitated and agglutinated. As a result, an area highly colored (in this embodiment, a spot) is generated at the bottom of the well 101. In this case, the sample is determined to be negative (that is, the sample is determined to contain no anti-HIV antibody 103).

As shown in Fig. 3, in a case where such an inhibitory substance 105 has been bound to the HIV antigen 102 carried on the inner surface of the well 101, the anti-IgG antibody 104 contained in the reagent fed into the well 101 is further bound to the inhibitory substance 105. Therefore, the colored particles 1 are adsorbed to the inner surface of the well 101 through the anti-IgG antibodies 104, the inhibitory substances 105 and the HIV antigens 102 with the colored particles 1 being dispersed in the liquid in the well 101. As a result, a negative liquid specimen is misjudged to be positive in spite of the fact that such an anti-HIV antibody is not present.

However, in the present invention, since the binding of the inhibitory substance 105 to the HIV antigen 102 is prevented due to the protein 106, it is possible to reliably prevent a mistaken determination. This makes it possible to determine the presence or absence of an anti-HIV antibody 103 in a sample, that is, to determine whether or not a living body is infected with HIV with accuracy.

Among various samples, particularly blood serum contains a great amount of the inhibitory substance 105. Therefore, the present invention is particularly suitable for use in determination of the presence or absence of an anti-HIV antibody in blood serum as a sample.

Further, in the present invention, since the precipitation inhibitory action of the inhibitory substance 105 is inhibited by the action of the protein 106, it is possible to eliminate the operation of cleaning for removing the inhibitory substance 105 from the well 101, thereby improving operating efficiency and avoiding the risk that an operator becomes infected with HIV due to a liquid specimen or the like spattering around the operator during the operation of cleaning. Therefore, it is possible to ensure a high degree of security.

Although the determination method and determination kit of the present invention have been described above, the present invention is not limited thereto.

For example, the determination method of the present invention may further contain any one or two or more steps, if necessary.

### Example

Hereinbelow, an actual example of the present invention will be described.

### 1. Preparation of a microplate

First, a microplate containing 96 wells having a v-shaped bottom (capacity: 0.3 mL) was prepared. Then, the mixture of the following 6 kinds of solutions of HIV antigens was fed into each of the wells (50 µL/well) of the microplate to allow the inner surface of each well to carry the 6 kinds of HIV antigens.

Recombinant HIV-1 IIIB gag p24 (manufactured by ImmunoDiagnostics): 1 µg/mL

Recombinant virus protein HIV-1 gp120 (manufactured by Advanced Biotechnologies): 0.5 µg/mL

Recombinant HIV-2 env(gp36) (manufactured by Virogen): 1 µg/mL

Recombinant HIV-1 env(gp41-gp120) (manufactured by Virogen): 1µg/mL

Recombinant HIV-1 p17/24/gp120·gp41 (manufactured by Virogen): 1 µg/mL

Recombinant HIV-1/-2 env (manufactured by Virogen): 1 µg/mL

### 2. Determination

### (Example 1A)

<1> First, an antagonist was prepared by dissolving casein as protein and polyoxyethylene(20)-sorbitan-monolaurate ("Tween 20" manufactured by Wako Pure Chemical Industries) as a nonionic surfactant in a potassium dihydrogen phosphate-disodium hydrogen phosphate buffered saline solution so that the concentrations thereof were 3.4 mg/mL and 0.24 vol%, respectively.
<2> Then, negative liquid specimens were prepared by diluting previously prepared negative blood serums (samples) containing no anti-HIV antibody with the antagonist 100-fold. Positive liquid specimens were also prepared by diluting previously prepared positive blood serums (samples) containing an anti-HIV antibody with the antagonist 100-fold. Further, a potassium dihydrogen phosphate-disodium hydrogen phosphate buffered saline solution was prepared as a reference solution.
   It is to be noted that the negative blood serums used here had been determined to contain no anti-HIV antibody and the positive blood serums had been determined to contain an anti-HIV antibody by ELISA.
<3> Next, each of the negative liquid specimens, the positive liquid specimens and the reference solution was fed into the wells in an amount of 0.05 mL per well. As a result, the amount of casein added per µg of the HIV antigen was 0.618 mg.
   It is to be noted that the amount of casein added per µg of the HIV antigen can be calculated as follows.
   Amount of casein per well = 3.4 mg/mL × 0.05 mL = 0.17 mg
   Concentration of HIV antigen in mixture fed into well = 5.5 µg/mL
   Amount of HIV antigen per well = 5.5 µg/mL × 50 µL = 0.275 µg
   Amount of casein per µg of HIV antigen per well = 0.17 mg ÷ 0.275 µg = 0.618 mg
   Then, a reagent containing nylon beads coated with hydroxyapatite (blue colored particles) carrying an antibody (anti-IgG antibody) to be bound to an anti-HIV antibody was fed into the wells.
   It is to be noted that the amount of the nylon beads coated with hydroxyapatite contained in the reagent was 0.1 wt%. The average particle diameter and the specific gravity of the nylon beads coated with hydroxyapatite were 5.8 µm and 1.13 g/cm³, respectively.
<4> Next, the motion and state of the nylon beads coated with hydroxyapatite in each of the wells was observed.

### (Comparative Example 1A)

The motion and state of the nylon beads coated with hydroxyapatite was observed in the same manner as in the Example 1A except that negative and positive liquid specimens were prepared by diluting negative and positive blood serums with a potassium dihydrogen phosphate-disodium hydrogen phosphate buffered saline solution 100-fold in the step <2>, respectively.

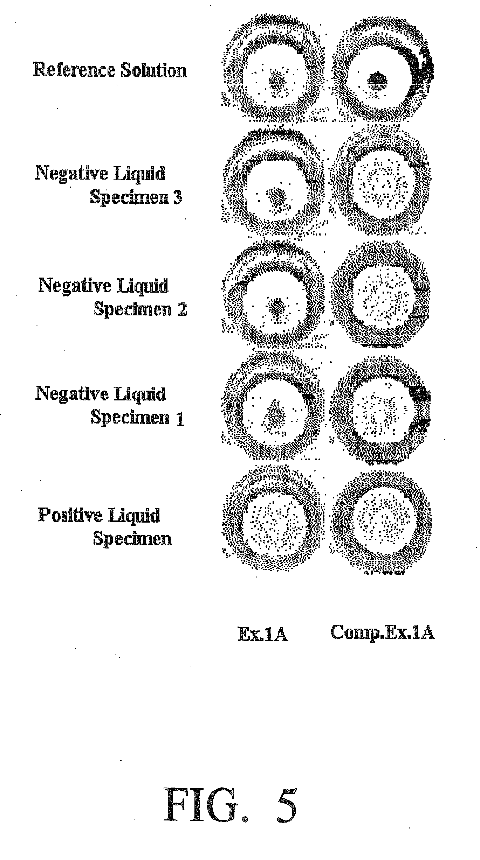

The results are shown in Fig. 5.

As shown in Fig. 5, in the Example 1A, all the negative liquid specimens showed results that should be determined to be negative, and all the positive liquid specimens showed results that should be determined to be positive.

On the other hand, in the Comparative Example 1A, all the negative and positive liquid specimens showed results that should be determined to be positive.

Further, in the Example 1A and Comparative Example 1A, it was confirmed that the nylon beads coated with hydroxyapatite were precipitated and aggregated in the reference solution. This result shows blood serum contains an inhibitory substance which interferes with the precipitation and aggregation of the nylon beads coated with hydroxyapatite.

### (Example 1B)

The motion and state of the nylon beads coated with hydroxyapatite was observed in the same manner as in the Example 1A except that negative liquid specimens were only used as a liquid specimen.

### (Example 2B)

The motion and state of the nylon beads coated with hydroxyapatite was observed in the same manner as in the Example 1B except that chymotrypsinogen A was used instead of casein.

### (Example 3B)

The motion and state of the nylon beads coated with hydroxyapatite was observed in the same manner as in the Example 1B except that lactoferrin was used instead of casein.

### (Example 4B)

The motion and state of the nylon beads coated with hydroxyapatite was observed in the same manner as in the Example 1B except that albumin was used instead of casein.

### (Example 5B)

The motion and state of the nylon beads coated with hydroxyapatite was observed in the same manner as in the Example 1B except that ovalbumin was used instead of casein.

### (Comparative Example 1B)

The motion and state of the nylon beads coated with hydroxyapatite was observed in the same manner as in the Example 1B except that negative liquid specimens were prepared by diluting negative blood serums with a potassium dihydrogen phosphate-disodium hydrogen phosphate buffered saline solution 100-fold in the step <2>.

### (Comparative Example 2B)

The motion and state of the nylon beads coated with hydroxyapatite was observed in the same manner as in the Example 1B except that tylosin was used instead of casein.

### (Comparative Example 3B)

The motion and state of the nylon beads coated with hydroxyapatite was observed in the same manner as in the Example 1B except that myoglobin was used instead of casein.

It is to be noted that tylosin and myoglobin have an affinity for the above-mentioned inhibitory substance lower than that of the HIV antigen.

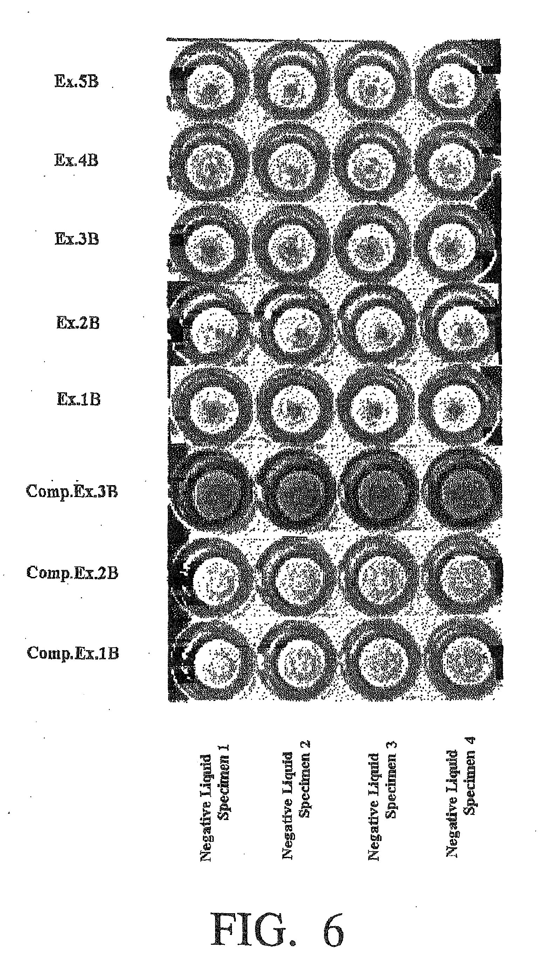

The results are shown in Fig. 6.

As shown in Fig. 6, in each of the Examples, all the negative liquid specimens showed results that should be determined to be negative.

Particularly, in the Example 1B (casein), the Example 3B (lactoferrin) and the Example 5B (ovalbumin), it was confirmed that the blue colored particles (the nylon beads coated with hydroxyapatite) were precipitated and aggregated in the negative liquid specimens more clearly.

On the other hand, in each of the Comparative Examples, all the negative liquid specimens showed results that should be determined to be positive.

It is to be noted that the motion and state of the nylon beads coated with hydroxyapatite was observed in the same manner as in the Examples except that casein, ovalbumin, albumin, lactoferrin and chymotrypsinogen A were arbitrarily combined to carry out the same determination mentioned above. The determination results were substantially the same as those of the Examples.

### Effect of the Invention

According to the present invention, it is possible to determine the presence or absence of an anti-HIV antibody in a sample with accuracy even if an operation of cleaning is omitted For this reason, the operation for determining can be carried out safely, easily and efficiently.

Finally, it is also to be understood that the present disclosure relates to subject matter contained in Japanese Patent Application No. 2005-306419 (filed on October 20, 2005) which is expressly incorporated herein by reference in its entirety.

## Claims

1. A determination method for determining whether or not a living body is infected with HIV (Human Immunodeficiency Virus) using a reagent containing particles carrying an anti-IgG antibody, comprising:
preparing a well carrying a HIV antigen on an inner surface thereof;
feeding a liquid specimen containing a sample obtained from the living body into the well, wherein the sample also containing an inhibitory substance which is to be bound to the HIV antigen and the anti-IgG antibody to interfere with the precipitation of the particles in the well;
feeding the reagent containing the particles into the well; and
determining that in a case where the anti-IgG antibody is bound to the anti-HIV antibody which has been bound to the HIV antigen so that the particles are dispersed in the well, the sample is positive, while that in a case where the particles are precipitated and agglutinated in the well, the sample is negative;
wherein before feeding the liquid specimen into the well, an antagonist containing a protein having an affinity for the inhibitory substance higher than that of the HIV antigen is added to the liquid specimen.

2. The determination method as claimed in claim 1, wherein the protein is at least one selected from the group comprising casein, ovalbumin, albumin, lactoferrin and chymotrypsinogen.

3. The determination method as claimed in claim 1, wherein the particles are colored.

4. The determination method as claimed in claim 1, wherein the specific gravity of the particles is larger than that of a liquid present in the well in the determining Step.

5. The determination method as claimed in claim 1, wherein the antagonist further contains a nonionic surfactant.

6. The determination method as claimed in claim 1, wherein the bottom of the well is made convergent.

7. The determination method as claimed in claim 1, wherein the sample is blood serum, and the liquid specimen is prepared by diluting the blood serum 2- to 1000-fold.

8. A determination kit to be used for the determination method defined by claim 1, comprising:
a plate having a plurality of wells each carrying the HIV antigen on an inner surface thereof;
a reagent containing the particles; and
an antagonist containing the protein.
